(19) Europäisches Patentamt
European Patent Office
Office européen des brevets

(11) **EP 4 513 180 A1**

(12) **EUROPEAN PATENT APPLICATION**
published in accordance with Art. 153(4) EPC

(43) Date of publication:
**26.02.2025 Bulletin 2025/09**

(21) Application number: **23791670.5**

(22) Date of filing: **04.04.2023**

(51) International Patent Classification (IPC):
**G01N 27/02** (2006.01)   **C12M 1/34** (2006.01)

(52) Cooperative Patent Classification (CPC):
**C12M 1/34; G01N 27/02**

(86) International application number:
**PCT/JP2023/013967**

(87) International publication number:
**WO 2023/204019 (26.10.2023 Gazette 2023/43)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **22.04.2022 JP 2022070705**

(71) Applicant: **SCREEN Holdings Co., Ltd.
Kyoto-shi, Kyoto 602-8585 (JP)**

(72) Inventor: **NAKAJIMA Shun
Kyoto-shi, Kyoto 602-8585 (JP)**

(74) Representative: **Goddar, Heinz J.
Boehmert & Boehmert
Anwaltspartnerschaft mbB
Pettenkoferstrasse 22
80336 München (DE)**

(54) **IMPEDANCE MEASUREMENT APPARATUS AND IMPEDANCE MEASUREMENT METHOD**

(57)    A technique is provided that allows use of a multielectrode array device to measure the impedances of biological samples. An impedance measurement apparatus (1) includes a measurement container (10), a plurality of first electrodes (31), a second electrode (32), a third electrode (33), a voltage application circuit (43), a current detection circuit (45), and a voltage detection circuit (47). The first electrodes (31) are arranged in an array on the bottom surface of the measurement container (10). The second electrode (32) and the third electrode (33) are located inside the measurement container (10). The voltage application circuit (43) applies a voltage between each first electrode (31) and the second electrode (32). The current detection circuit (45) detects current flowing through each first electrode (31). The voltage detection circuit (47) detects a voltage between each first electrode (31) and the third electrode (33).

Fig. 1

EP 4 513 180 A1

**Description**

[Technical Field]

The subject matter disclosed in the specification of the present invention relates to an impedance measurement apparatus and an impedance measurement method.

[Background Art]

In the field of electrophysiology, analysis is conducted on the activities of a single or a group of cellular ion channels. Examples of known ion channel analyzers include intracellular action potential detectors using a patch-clamp method and extracellular action potential detectors using a multielectrode array (MEA) device. For example, Patent Literature (PTL) 1 discloses a multielectrode array device.

[Citation List]

[Patent Literature]

PTL 1: Japanese Unexamined Patent Application Publication (Translation of PCT Application) No. 2016-529889

[Summary of Invention]

[Technical Problem]

For example, there may be situations in which the impedances of target biological samples (including cells and biological slices) are measured in order to easily measure changes in cell shape or the density of cells. However, multielectrode array devices have conventionally been used for only detection of extracellular action potentials, and technology for measuring the impedances of biological samples by using a multielectrode array device has not yet been found.

It is an object of the present invention to provide a technique that allows use of a multielectrode array device to measure the impedances of biological samples.

[Solution to Problem]

To solve the problem described above, a first aspect is an impedance measurement apparatus that includes a measurement container, a plurality of first electrodes arranged in an array on a bottom surface of the measurement container, a second electrode and a third electrode that are located inside the measurement container, a voltage application circuit that applies a voltage between each of the first electrodes and the second electrode, a current detection circuit that detects current flowing through each of the first electrodes, and a voltage detection circuit that detects a voltage between each of the first electrodes and the second electrode.

A second aspect is the impedance measurement apparatus according to the first aspect, in which the third electrode is located on the bottom surface.

A third aspect is the impedance measurement apparatus according to the first or second aspect that further includes an electrode selection circuit that selects one first electrode that is to be connected to the current detection circuit, from among the plurality of first electrodes.

A fourth aspect is the impedance measurement apparatus according to any one of the first to third aspects, in which the current detection circuit is capable of outputting a voltage responsive to the current. The impedance measurement apparatus further includes a differential-signal detection circuit that detects a difference between an alternating voltage applied by the voltage application circuit and a voltage output from the current detection circuit.

A fifth aspect is the impedance measurement apparatus according to any one of the first to fourth aspects that further includes a calculation unit that calculates an impedance in accordance with the current detected by the current detection circuit and the voltage detected by the voltage detection circuit.

A sixth aspect is an impedance measurement method that includes a) applying an alternating voltage between at least one of a plurality of first electrodes and a second electrode, the plurality of first electrodes being arranged in an array on a bottom surface of a measurement container, the second electrode being located inside the measurement container, b) detecting current flowing through the first electrode(s) while the alternating voltage is applied by the operation a), c) detecting a voltage between the first electrode(s) and a third electrode while the alternating voltage is applied by the operation a), and d) calculating an impedance of a target object in accordance with the current detected by the operation b) and the voltage detected by the operation c).

[Advantageous Effects of Invention]

The impedance measurement apparatus according to the first aspect is capable of measuring the impedance of a target object by a three-terminal method using the first electrodes arranged in an array on the bottom surface of the measurement container.

The impedance measurement apparatus according to the second aspect decreases in size because the first electrodes and the third electrode are concentrated on the bottom surface.

The impedance measurement apparatus according to the third aspect is capable of measuring the impedance by using one first electrode selected from among the plurality of first electrodes.

The impedance measurement apparatus according to the fourth aspect is capable of calculating the impedance of the target object in accordance with the phase difference between the alternating voltage and the current.

The impedance measurement apparatus according to the fifth aspect is capable of calculating an impedance.

[Brief Description of Drawings]

[Fig. 1] Fig. 1 is a diagram schematically showing a configuration of an impedance measurement apparatus according to an embodiment.
[Fig. 2] Fig. 2 is a top view of a measurement container shown in Fig. 1.
[Fig. 3] Fig. 3 is a diagram showing an equivalent circuit in the case of measuring an impedance.

[Description of Embodiment]

Hereinafter, one embodiment of the present invention will be described with reference to the accompanying drawings. Note that constituent elements described in the embodiment are merely examples and do not intend to limit the scope of the present invention. To facilitate understanding of the drawings, the dimensions or number of constituent elements may be illustrated in an exaggerated or simplified manner as necessary.

1. First Embodiment

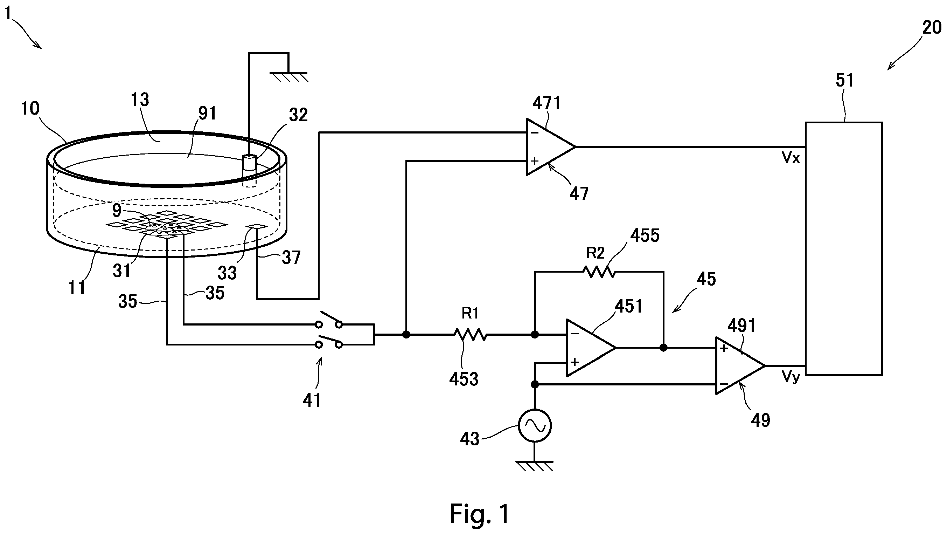

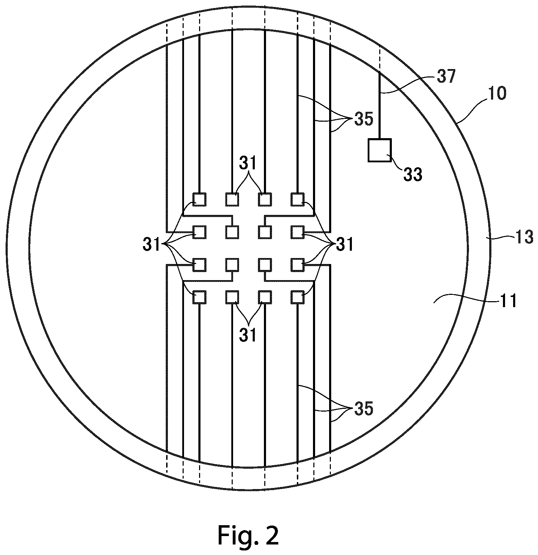

Fig. 1 is a diagram schematically showing a configuration of an impedance measurement apparatus 1 according to an embodiment. Fig. 2 is a top view of a measurement container 10 shown in Fig. 1. As shown in Fig. 1, the impedance measurement apparatus 1 includes the measurement container 10 and a measuring unit 20.

The measurement container 10 is a container for measuring the impedances of cells 9 serving as a target object. A cell suspension that contains the cells 9 is dropped into the measurement container 10. The measurement container 10 has a bottom 11 and a side wall 13 as shown in Fig. 1. The bottom 11 expands in a disk-like shape along a horizontal plane. The side wall 13 extends upward in a cylindrical shape from the edge of the bottom 11. The bottom 11 is an example of a "measurement plate." The bottom 11 has an upper surface that corresponds to the bottom surface of the measurement container 10 into which the cell suspension is dropped. Note that an object to be measured by the measurement container 10 is not limited to the cells 9 and may, for example, be a sample of biological tissue slices.

The measurement container 10 includes a plurality of first electrodes 31, a second electrode 32, a third electrode 33, a plurality of first wires 35, and a second wire 37. The first electrodes 31, the third electrode 33, the first wires 35, and the second wire 37 are located on the upper surface of the bottom 11 (the bottom surface of the measurement container 10). Each first wire 35 and the second wire 37 are covered with an insulator (e.g., light-sensitive polyimide). For example, each first electrode 31, the third electrode 33, each first wire 35, and the second wire 37 may be formed by photolithography on the upper surface of the bottom 11.

The first electrodes 31 are arranged in an array. The measurement container 10 is a multielectrode array (or microelectrode array) device that includes the plurality of fine first electrodes 31 arranged in an array. In the examples shown in Figs. 1, 2, and 3, sixteen first electrodes 31 are arranged in a matrix with four rows and four columns. Note that the number and layout of the first electrodes 31 may be set freely. Preferably, ten or more first electrodes 31 may be arranged. The first electrodes 31 have a square shape when viewed from above. It is, however, noted that the shape of the first electrodes 31 may be any other shape different from the square shape, such as a polygonal shape or a circular shape.

The second electrode 32 is located inside the measurement container 10. For example, the second electrode 32 may have a stick-like shape, and at least a part (e.g., the lower end portion) is immersed in a liquid 91 that is injected into the measurement container 10.

The third electrode 33 is located radially outward of and away from the first electrodes 31. The first electrodes 31 and the third electrode 33 are isolated from each other. In the examples shown in Figs. 1 and 2, the third electrode 33 has a square shape when viewed from above. It is however, noted that the shape of the third electrode 33 may be any other shape different from the square shape, such as a polygonal shape or a circular shape.

Each first wire 35 is electrically connected to a corresponding one of the first electrodes 31. Fig. 1 shows two first cables 35 connected to two first electrodes 31 and does not show first cables 35 that are connected to the other first electrodes 31. Each first cable 35 extends to the outside of the measurement container 10. The second wire 37 is electrically connected to the second electrode 33. The second wire 37 extends from the third electrode 33 to the outside of the measurement container 10. As shown in Fig. 1, each first wire 35 and the second wire 37 are electrically connected to the measuring unit 20 arranged outside the measurement container 10.

As shown in Fig. 1, the measuring unit 20 includes an electrode selection circuit 41, a voltage application circuit 43, a current detection circuit 45, a voltage detection circuit 47, a differential-signal detection circuit 49, and a calculation unit 51.

The electrode selection circuit 41 is a circuit for selecting one first electrode 31 that is to be electrically connected to the current detection circuit 45 and the voltage detection circuit 47, from among the plurality of first electrodes 31. As shown in Fig. 1, the electrode selection circuit 41 includes a plurality of switches that open and close circuits that connect each of the first electrodes 31 to the current detection circuit 45. The switching operation of the electrode selection circuit 41 may be performed under the control of the calculation unit 51.

The voltage application circuit 43 is electrically connected to the first electrodes 31 via the electrode selection circuit 41. The voltage application circuit 43 applies an alternating voltage (alternating signal) of a predetermined frequency between the first electrode 31 and the second electrode 32. In the example shown in Fig. 1, the voltage application circuit 43 is connected to the non-inverting input terminal (+) of an operational amplifier 451, which will be described later, of the current detection circuit 45. The voltage application circuit 43 is also connected to the inverting input terminal (-) of an operational amplifier 491, which will be described later, of the differential-signal detection circuit 49.

The current detection circuit 45 is electrically connected to the electrode selection circuit 41. The current detection circuit 45 detects current flowing through a first electrode 31 selected by the electrode selection circuit 41.

As shown in Fig. 1, the current detection circuit 45 includes the operational amplifier 451, a first resistor 453, and a second resistor 455. The non-inverting input terminal (+) of the operational amplifier 451 is electrically connected to the voltage application circuit 43. The inverting input terminal (-) of the operational amplifier 451 is electrically connected to the electrode selection circuit 41. The first resistor 453 is located between the electrode selection circuit 41 and the inverting input terminal (+) of the operational amplifier 451. One end of the second resistor 455 is connected between the differential-signal detection circuit 49 and an output terminal of the operational amplifier 451. The other end of the second resistor 455 is connected between the first resistor 453 and the inverting input terminal (-) of the operational amplifier 451.

The voltage detection circuit 47 detects a voltage Vx between the third electrode 33 and one first electrode 31 selected by the electrode selection circuit 41. As shown in Fig. 1, the voltage detection circuit 47 includes an operational amplifier 471. The non-inverting input terminal (+) of the operational amplifier 471 is connected between the electrode selection circuit 41 and the first resistor 453 and is thus electrically connected to one first electrode 31 via the electrode selection circuit 41. The inverting input terminal (-) of the operational amplifier 471 is electrically connected to the third electrode 33.

The differential-signal detection circuit 49 detects a phase difference between the alternating voltage applied by the voltage application circuit 43 and the current detected by the current detection circuit 45. As shown in Fig. 1, the differential-signal detection circuit 49 includes the operational amplifier 491. The non-inverting input terminal (+) of the operational amplifier 491 is connected to the output terminal of the operational amplifier 451 of the current detection circuit 45. The inverting input terminal (-) of the operational amplifier 491 is connected to the voltage application circuit 43.

The calculation unit 51 includes a storage device, a processing circuit, an input device, and an output device. The storage device may be configured as, for example, memory (storage medium) such as a hard disk drive (HDD), random-access memory (RAM), read-only memory (ROM), flash memory, volatile or nonvolatile semiconductor memory, a magnetic disk, a flexible disk, an optical disk, a compact disc, a minidisc, or a DVD. The processing circuit may be configured as, for example, a central processing unit (CPU) that executes programs stored in the storage device. The input device may be configured as, for example, a device that is capable of inputting information, such as a mouse, a keyboard, a touch panel, or a variety of switches. The output device may be configured as, for example, a device that is capable of outputting information, such as a display, a liquid crystal display, or a lamp.

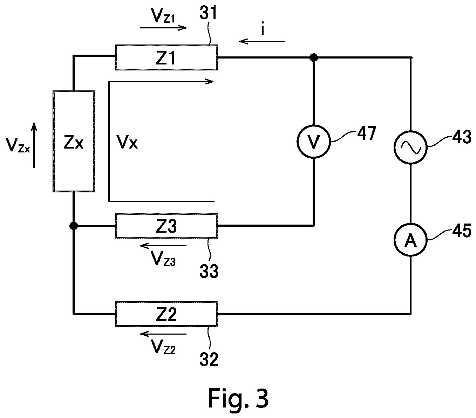

The calculation unit 51 calculates the impedance in accordance with the voltage Vx output from the voltage detection circuit 47 and a voltage Vy output from the differential-signal detection circuit 49. The procedure for calculating the impedance is described with reference to Fig. 3.

Impedance Calculation

Fig. 3 is a diagram showing an equivalent circuit in the case of measuring the impedance. In the case where the measurement container 10 is used to measure the impedance of the cells 9, the measurement container 10 is placed on a horizontal base. Then, a cell suspension is dropped onto the upper surface of the bottom 11 of the measurement container 10 on which the first electrodes 31 are arranged. After the dropped cell suspension is left in the measurement container 10 for several minutes, a plurality of cells 9 are settled and form a cell layer on the bottom 11 of the measurement container 10. After the formation of the cell layer, the liquid 91 for measurement (e.g., culture medium) is injected into the measurement container 10. The liquid 91 comes in contact with the first electrodes 31, the second electrode 32, and the third electrode 33 so as to bring the first electrodes 31, the second electrode 32, and the third electrode 33 into conduction. Note that the second electrode 32 is grounded during impedance measurement as shown in Fig. 1.

In the following description, "Z1" represents the contact impedance of the first electrode 31, "Z2" represents the contact impedance of the second electrode 32, "Z3" represents the contact impedance of the third electrode 33, and "Zx" represents the impedance (target impedance) of a target object (cells) as shown in Fig. 3.

As shown in Fig. 3, when the voltage application circuit 43 has applied an alternating voltage, alternating current i flows through the first electrode 31. During the application of the alternating voltage from the voltage application circuit 43, potential differences caused by the contact impedances Z1, Z2, and Z3 and the impedance Zx are expressed respectively as "$V_{Z1}$", "$V_{Z2}$", "$V_{Z3}$", and "$V_{Zx}$".

The voltage detection circuit 47 detects the voltage Vx between the first electrode 31 and the third electrode 33. The voltage Vx is equal to a total value of $V_{Zx}$, $V_{Z1}$, and $V_{Z3}$. The voltage Vx is also expressed by the following Expression (1), using the current i, the contact impedances Z1 and Z3, and the target impedance Zx.

$$Vx = V_{Zx} + V_{Z1} + V_{Z3} = (Zx + Z1 + Z3)*i \quad \cdot \cdot \cdot (1)$$

Here, increasing the input impedance of the operational amplifier 471 of the voltage detection circuit 47 (to, for example, 10 giga ohms (G$\Omega$) or higher) will make the current hard to flow into the voltage detection circuit 47. Accordingly, the potential difference $V_{Z3}$ caused at the third electrode 33 will become nearly zero ($V_{Z3} \fallingdotseq 0$). Thus, the voltage detection circuit 47 substantially measures a total value of $V_{Z1}$ and $V_{Zx}$ ($= V_{Z1} + V_{Zx}$) and can reduce the contact impedance Z3 of the third electrode 33. That is, the voltage Vx measured by the voltage detection circuit 47 is expressed by the following Expression (2)

$$Vx = V_{ZX} + V_{Z1} = (Zx + Z1)*i \quad \cdot \cdot \cdot (2)$$

Here, the alternating current i is expressed by the following Expression (3), where Vs represents the voltage applied by the voltage application circuit 43, and R1 represents the resistance value of the first resistor 453.

$$i = Vs/R1 \quad \cdot \cdot \cdot (3)$$

The voltage Vy output from the differential-signal detection circuit 49 is the difference between the applied voltage Vs and the voltage ($= R2*i$) output from the current detection circuit 45. That is, the voltage Vy is expressed by the following Expression (4).

$$Vy = (R2*i)\text{-}Vs \quad \cdot \cdot \cdot (4)$$

Since a signal phase difference can be detected by measuring the voltages Vx and Vy, the composite impedance of Zx + Z1 can be measured. The contact impedance Z1 is measured by conducting an impedance measurement in the presence of only the liquid 91 (i.e., no cells 9: Zx = 0). The target impedance Zx can be calculated by subtracting the measured contact impedance Z1 from the composite impedance of Zx + Z1.

As described above, the impedance measurement apparatus 1 is capable of measuring the impedance of the cells 9 as a target object, by measuring the current flowing through the first electrodes 31 serving as a multielectrode array. Accordingly, it is possible to measure not only conventional extracellular action potentials but also the impedance of the cells 9 by using the measurement container 10 serving as a multielectrode array device.

The impedance measurement apparatus 1 measures the impedance using a three-terminal method using three electrodes. This improves the accuracy of measuring the impedance of the cells 9 compared to using a two-terminal method.

2. Variations

While the embodiment has been described thus far, the present invention is not intended to be limited to the above-described embodiment.

For example, the second electrode 32 may be formed by photolithography on the surface of the side wall 13.

It is not essential to locate the third electrode 33 on the upper surface of the bottom 11 of the measurement container 10. For example, like the second electrode 32, the third electrode 33 may be formed in a stick shaped form and configured to be immersed in the liquid injected into the measurement container 10. Additionally, a plurality of third electrodes 33 may be arranged. In this case, a section circuit, which is not shown, may be used to select one of the third electrodes 33 that is connected to the voltage detection circuit 47.

While the invention has been shown and described in detail, the foregoing description is in all aspects for illustration purposes only and not limiting. It is therefore understood that numerous modifications and variations that are not illustrative above can be devised without departing from the scope of the invention. The configurations in the embodiments and variations described above may be appropriately combined or omitted as long as there are no mutual inconsistencies.

[Reference Signs List]

1      impedance measurement apparatus
10    measurement container
11    bottom
20    measuring unit
31    first electrode
32    second electrode
33    third electrode
41    electrode selection circuit
43    voltage application circuit
45    current detection circuit
47    voltage detection circuit
49    differential-signal detection circuit
51    calculation unit

**Claims**

1. An impedance measurement apparatus comprising:

   a measurement container;
   a plurality of first electrodes arranged in an array on a bottom surface of the measurement container;
   a second electrode and a third electrode that are located inside the measurement container;
   a voltage application circuit that applies a voltage between each of the first electrodes and the second electrode;
   a current detection circuit that detects current flowing through each of the first electrodes; and
   a voltage detection circuit that detects a voltage between each of the first electrodes and the third electrode.

2. The impedance measurement apparatus according to claim 1, wherein
   the third electrode is located on the bottom surface.

3. The impedance measurement apparatus according to claim 1 or 2, further comprising:
   an electrode selection circuit that selects one first electrode that is to be connected to the current detection circuit, from among the plurality of first electrodes.

4. The impedance measurement apparatus according to any one of claims 1 to 3, wherein

   the current detection circuit is capable of outputting a voltage responsive to the current,
   the impedance measurement apparatus further comprising:
   a differential-signal detection circuit that detects a difference between an alternating voltage applied by the voltage application circuit and a voltage output from the current detection circuit.

5. The impedance measurement apparatus according to any one of claims 1 to 4, further comprising:
a calculation unit that calculates an impedance in accordance with the current detected by the current detection circuit and the voltage detected by the voltage detection circuit.

6. An impedance measurement method comprising:

a) applying an alternating voltage between at least one of a plurality of first electrodes and a second electrode, the plurality of first electrodes being arranged in an array on a bottom surface of a measurement container, the second electrode being located inside the measurement container;
b) detecting current flowing through the first electrode(s) while the alternating voltage is applied by the operation a);
c) detecting a voltage between the first electrode(s) and a third electrode while the alternating voltage is applied by the operation a); and
d) calculating an impedance of a target object in accordance with the current detected by the operation b) and the voltage detected by the operation c).

Fig. 1

Fig. 2

Fig. 3

## INTERNATIONAL SEARCH REPORT

International application No.

**PCT/JP2023/013967**

### A. CLASSIFICATION OF SUBJECT MATTER

***G01N 27/02***(2006.01)i; ***C12M 1/34***(2006.01)i
FI: G01N27/02 D; C12M1/34

According to International Patent Classification (IPC) or to both national classification and IPC

### B. FIELDS SEARCHED

Minimum documentation searched (classification system followed by classification symbols)

G01N27/00-G01N27/10; G01N27/14-G01N27/24; C12M1/00-C12M1/42; G01N33/48-G01N33/98; G01R27/00-G01R27/32;C12Q1/00-C12Q1/70

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | WO 2020/189172 A1 (SCREEN HOLDINGS CO., LTD.) 24 September 2020 (2020-09-24) paragraphs [0029]-[0083], fig. 1-4 | 1-3, 5-6 |
| A | | 4 |
| Y | JP 2021-083369 A (RITSUMEIKAN) 03 June 2021 (2021-06-03) paragraphs [0020]-[0030], fig. 1, 2 | 1-3, 5-6 |
| Y | JP 2021-164460 A (NOVASCAN, INC.) 14 October 2021 (2021-10-14) claim 1, paragraphs [0002], [0030], [0031], [0036] | 1-3, 5-6 |
| Y | JP 2000-206162 A (NTT POWER & BUILDING FACILITIES INC.) 28 July 2000 (2000-07-28) paragraph [0002], fig. 4 | 1-3, 5-6 |
| A | US 2014/0284221 A1 (IMEC) 25 September 2014 (2014-09-25) entire text | 1-6 |

☐ Further documents are listed in the continuation of Box C.      ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **28 April 2023** | **16 May 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2023/013967**

| Patent document cited in search report | | | Publication date (day/month/year) | Patent family member(s) | | | Publication date (day/month/year) |
|---|---|---|---|---|---|---|---|
| WO | 2020/189172 | A1 | 24 September 2020 | JP 2020-150852 A<br>paragraphs [0016]-[0070], fig. 1-4 | | | |
| JP | 2021-083369 | A | 03 June 2021 | (Family: none) | | | |
| JP | 2021-164460 | A | 14 October 2021 | WO 2018/136865 A1<br>claim 1, paragraphs [0002], [0036], [0037], [0042] | | | |
| JP | 2000-206162 | A | 28 July 2000 | (Family: none) | | | |
| US | 2014/0284221 | A1 | 25 September 2014 | EP 2781906 A2 | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016529889 W